# EUROPEAN PATENT APPLICATION

(11) **EP 0 522 596 A1**
(43) Date of publication of application: **13.01.1993**
(21) Application number: 92113164.5
(22) Date of filing: 03.04.1990
(51) Int. Cl.: A61K 37/24, C07K 7/06, C07K 7/10

(54) **Method and means for inducing constriction of the pupil in the eye**

(30) Priority: 03.04.1989 SE 8901149
(62) Divisional of application: 90906391.9
(71) Applicant: Kabi Pharmacia AB, S-751 82 Uppsala (SE)
(72) Inventor: Bill, Anders, S-756 45 Uppsala (SE)

(57) **Abstract**

The use of cholecystokinin and derivatives of cholecystokinin for inducing miosis in the eye (pupil constriction) after certain types of examinations and operations. The invention moreover also comprises ophthalmological compositions containing an active amount of cholecystokinin or of its derivatives.

## Description

The invention relates to the use of cholecystokinin and derivatives of cholecystokinin for inducing miosis in the eye (constriction of the pupil) after certain types of examinations and operations. Also, the invention relates to ophthalmological compositions containing an active amount of cholecystokinin or its derivatives.

The size of the pupil in the eye is governed by two muscles in the iris, opposite in character in respect of their mode of action. One of these muscles when undergoing contraction will produce a dilatation of the pupil (dilatator muscle); it is controlled by nerve fibers from the sympathetic nervous system. The other muscle (sphincter muscle situated near the iris edge region, i.e. near the pupil) will cause a diminution of the pupil. This muscle is governed by parasympathetic nerves which utilize acetylcholine as transmitter.

It has been known for a long time that constriction of the pupil, i.e. miosis, can be caused by mechanisms other than acetylcholine release. In particular this occurs as part of the eye's response to various kinds of irritation. Such miosis cannot be prevented by antagonists of acetylcholine such as e.g. atropine. Clinically miosis of the type produced by irritation often implies substantial complications, e.g. in intraocular surgery. In cases of uveites (inflammation of the uvea), especially iritis, this type of miosis will sometimes give rise to very undesirable synechiae between the iris and the lens. Miosis caused by irritation has been studied thoroughly in experimental animals; as reported in a paper from my laboratory (Bill et al., 1979) it was found that such irritation causes a peptide very similar to substance P to be released in the eye. When synthetically produced substance P was injected into the anterior chamber this produced a substantial contractive response of the sphincter muscle of the iris, thus suggesting that this miosis is brought about by the said peptide or a closely related substance.

It should be noted however that there are considerable variations from species to species as regards pupillary responses to substance P - despite the fact that this neuropeptide has been detected immunohistochemically in the eyes of many species, including primates. It has been found inter alia that this response is totally absent in primates (Mandahl et al., 1980) and that therefore in primates irritation-caused miosis must be due to some other substance. Thus, the effect of certain neuropeptides is dependent on the presence or absence of receptors in the tissue innervated by sensory nerves, and such presence or absence of receptors differs very much between animal species. For this reason it is entirely impossible to predict whether conditions valid in e.g. rabbit iris will be valid to the iris of monkeys or humans, and vice versa, even though the same peptide may have been identified in sensory nerves of the iris in different species.

In the rabbit, irritation will cause substance P to be released from the sensory nerve fibers in the iris. The iris sensory nerves contain a number of other potential transmitters, one of these being cholecystokinin (CCK) or a substance related to CCK; see Kuwayama et al., 1987. In my laboratory we have found that CCK does not induce miosis in the rabbit, despite its presence in sensory nerves.

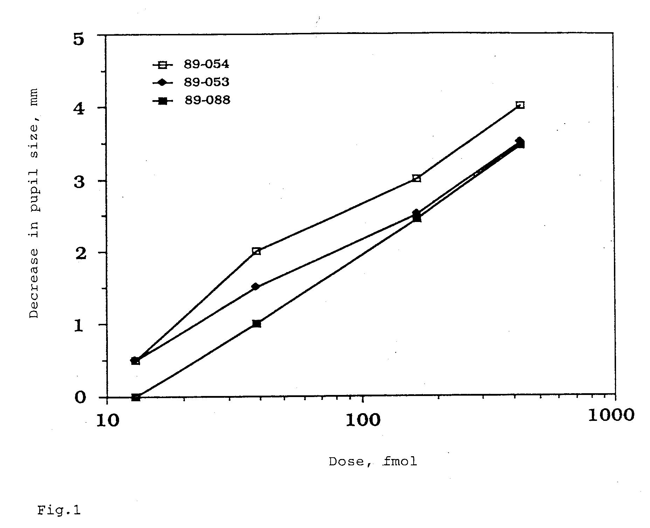

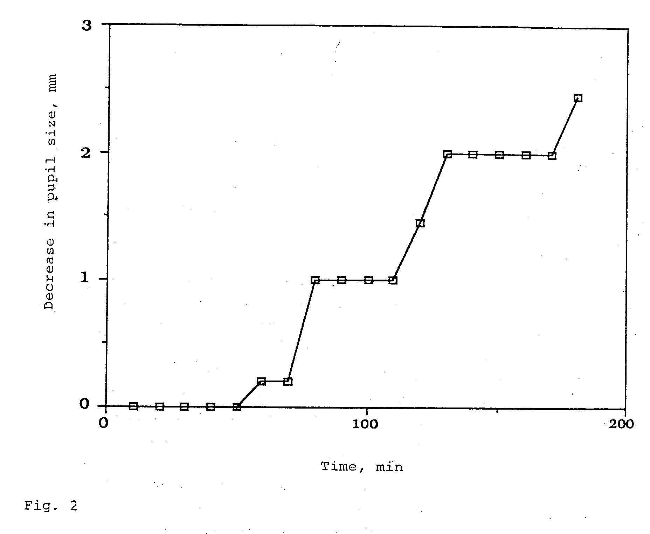

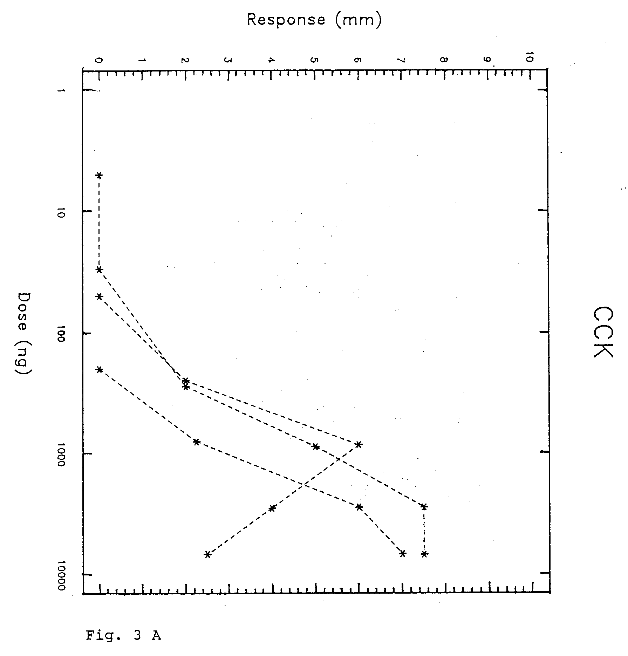

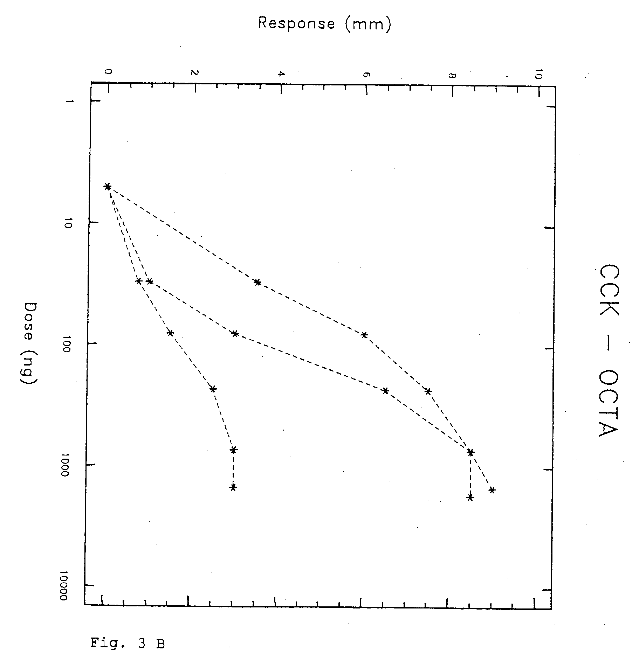

While looking for the substance that is responsible in primates for the miosis response to local irritation we have unexpectedly found in my laboratory that CCK has a very potent miotic effect in monkeys (Macaca fascicularis). A dose of about 0.21 pmol (≈800 picograms) injected into the anterior chamber will give a half-maximal effect; a nearly maximal effect is seen at 1 pmol (≈3.9 nanograms). Fragments of the C-terminal portion of the peptide are also active: The sulfated terminal 8-aminoacid sequence is about 10 times more potent than the whole CCK molecule (Figure 1 which shows how in three monkey eyes injection of CCK-8 into the anterior chamber will affect the size of the pupil). This fragment results in a decrease of pupil size also upon administration in the form of eye drops (Figure 2 which shows how corneal application of CCK-8 will affect pupil size). The amino acid sequence of CCK, its C-terminal octapeptide, and its C-terminal tetrapeptide are as follows: 
CCK-33

Lys-Ala-Pro-Ser-Gly-Arg-Val-Ser-Met-Ile-Lys-Asn-Leu-Gln-Ser-
-Leu-Asp-Pro-Ser-His-Arg-Ile-Ser-Asp-Arg-Asp-Tyr-(SO3H)-Met-
Gly-Trp-Met-Asp-Phe-NH2

CCK 26-33 (octapeptide)

Asp-Tyr-(SO3H)-Met-Gly-Trp-Met-Asp-Phe-NH2

CCK 30-33 (tetrapeptide)

Trp-Met-Asp-Phe-NH2
In other experiments, in vitro, we have shown that CCK, its C-terminal octapeptide but not its C-terminal tetrapeptide will contract the iris sphincter muscle isolated from monkey (Figure 3 which shows a cumulated dose-response curve of the cholecystokinin effect on monkey iris in vitro (3A) and cumulated dose-response curve of the cholecystokinin C-terminal octapeptide effect (26-33) on monkey iris in vitro (3B).

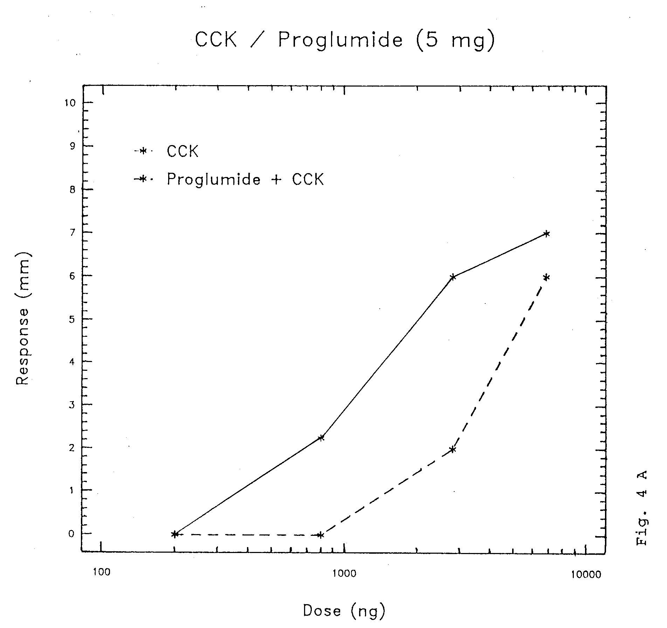

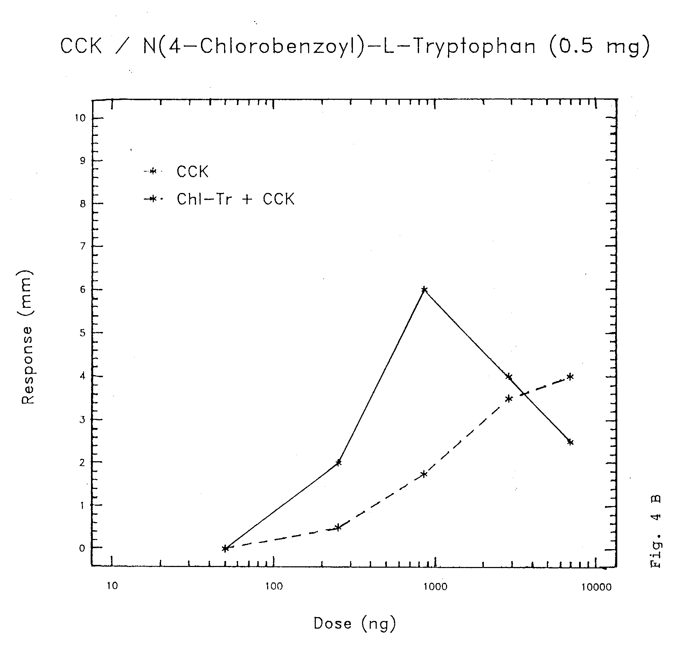

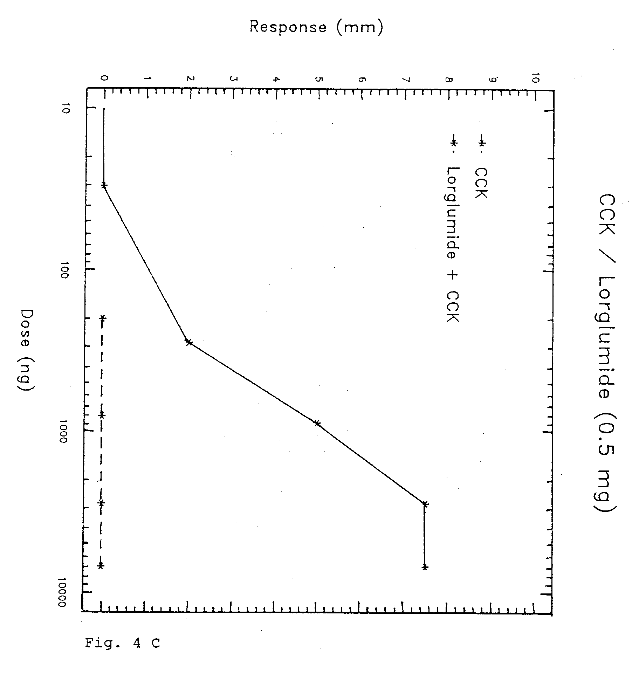

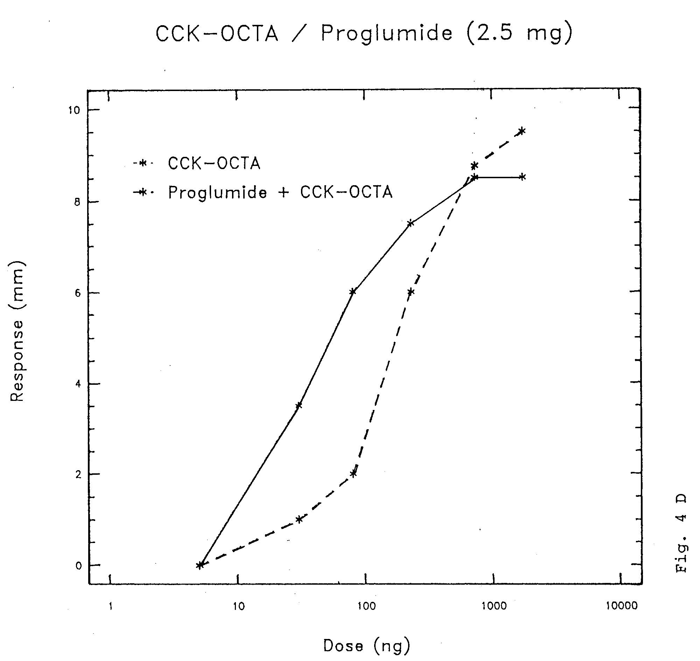

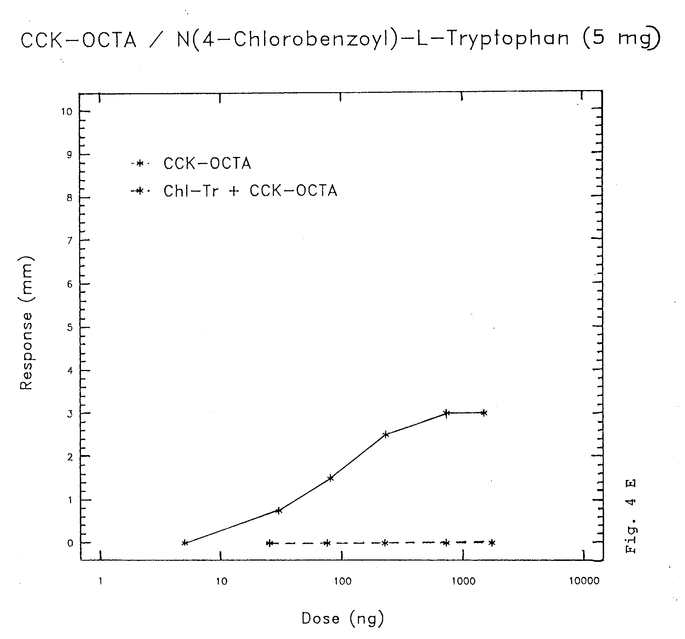

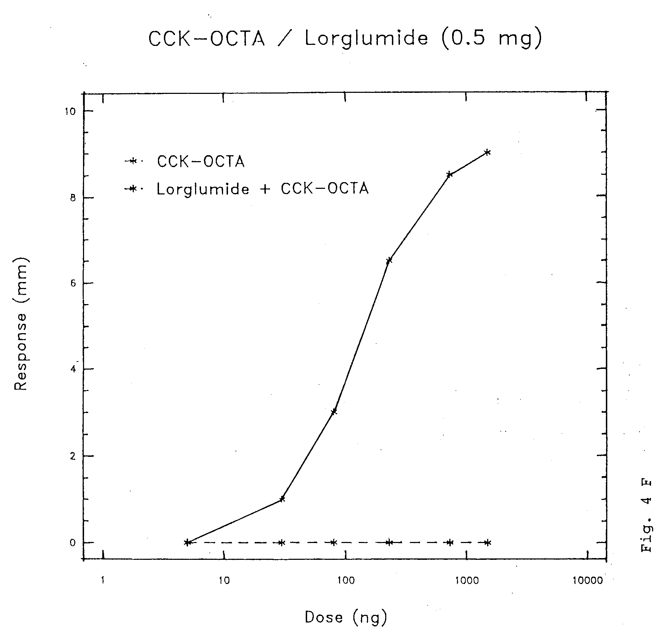

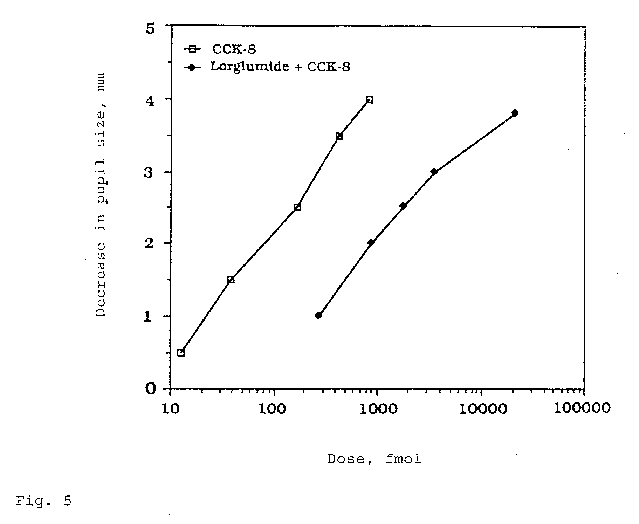

It appears that the C-terminal octapeptide is the more potent one of these two. Furthermore we have shown that several antagonists to CCK (CCK blockers) as e.g. CR 1409, also called "Lorglumide" (D,L-4-(3,4-dichlorobenzoylamino)-5-(dipentylamino)-5-oxo-pentanoic acid), Proglumide DL4-benzamido-N,N-dipropyl-glutaramic acid and N-(4-chlorobenzoyl)-L-tryptophan will effectively cause the dose-response curve of CCK and its C-terminal octapeptide to be shifted to the right, or will totally block the effect of CCK on isolated iris sphincter from monkey (Figure 4 which shows cumulated dose-response curves of CCK and of its C-terminal octapeptide with and without antagonists). This means that the CCK receptors on the smooth muscles of the iris sphincter are subject to direct competitive blockage. Experiments carried out in my laboratory moreover show that Lorglumide antagonizes CCK also in vivo with respect to the miosis response. Upon injection of 0.75 µg of Lorglumide into the anterior chamber the dose-response curve of CCK-8 was shifted to the right by more than one order of magnitude (Figure 5 which shows CCK-8 dose-response curves with and without pretreatment with the antagonist Lorglumide).

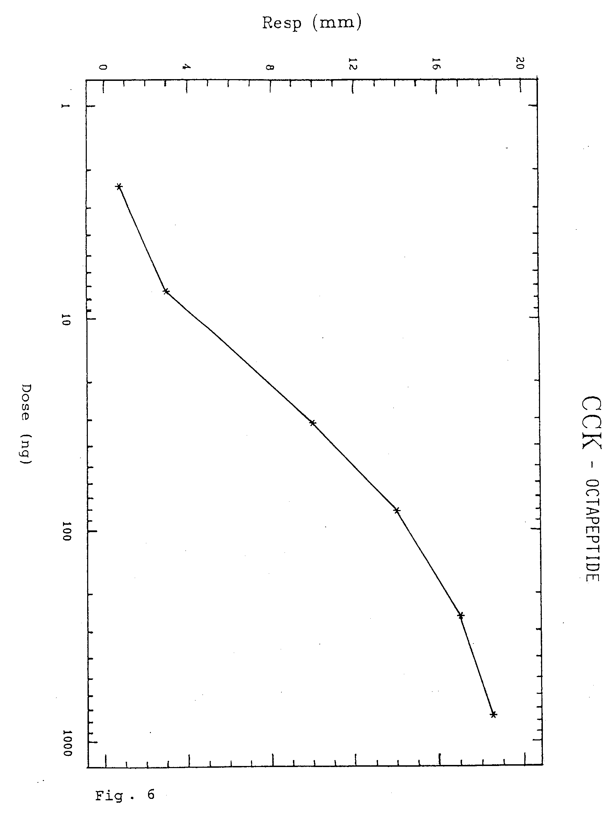

In addition, we have found that CCK has a direct effect on a receptor present on the sphincter muscle. This may be concluded from the fact that nerve blockade with tetrodotoxin and pretreatment with indomethacin do not cause a decrease in the miosis response. These experiments show that the receptors are not located on other nerves and that cyclo-oxygenase products of arachidonic acid metabolism are not involved in the miosis response to CCK. In addition an effect on isolated human pupil sphincter comparable to the in vitro effect on monkeys has also been found (Figure 6 which shows a cumulated dose-response curve of the CCK C-terminal octapeptide on the sphincter muscle of human iris in vitro), thus confirming that the same mechanism also exists in the human eye. Capsaicin (8-methyl-N-vanillyl-6-nonenamide) is a highly irritative substance which in the eye of the rabbit will produce miosis due to release of substance P from sensory nerves of the iris (Mandahl et al., 1984). Experiments that have now been carried out have revealed a miotic response to capsaicin in monkeys, although this is less consistent and less pronounced as compared to the response in rabbits. Very probably this response in the monkey is secondary to the release of a substance from the iridial sensory nerves.

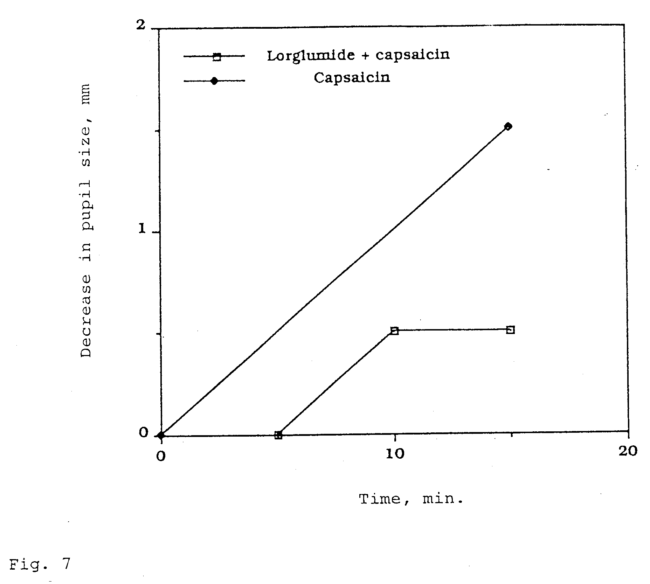

The nature of the substance is still unknown, but there is good reason to believe that this substance is CCK or a closely related substance with a terminal portion similar to the C-terminal portion of CCK. This assumption is supported by experiments with animals that have shown sensitivity to capsaicin. After pretreatment with Lorglumide, a CCK antagonist, most of the effect on the pupil was eliminated (Figure 7 which shows the effect of capsaicin on pupil size with and without pretreatment with 7.5 µg Lorglumide).

It now seems highly probable that the miosis seen in human beings in irritative conditions such as iritis, uveitis, and in cases of surgery in the anterior chamber, is due to the release of a substance similar to or consisting of CCK. Consequently it ought to be possible to prevent this miosis by means of CCK antagonists. Cholecystokinin antagonists have been subdivided into a number of different classes, i.e. such CCK antagonists that are derivatives of cyclic nucleotides and such that are derivatives of amino acids and C-terminal and N-terminal fragments of CCK. N₂,O₂-dibutyryl cyclic guanosine 3',5'-monophosphate is an example of cyclic nucleotide derivatives that have been described as having an antagonistic effect against CCK.

Proglumide, and (D,L-4(3,4-dichlorobenzoylamino)-5-(N-3-methoxypropyl-pentylamino)-5-oxo-pentanoic acid), also called Loxiglumide or CR-1505, as well as other derivatives of glutaramic acid have been found to have a very good inhibitory effect on CCK receptors. Lorglumide (D,L-4-(3,4-dichlorobenzoylamino)-5-(dipentylamino)-5-oxo-pentanoic acid, also called CR-1409, as well as other derivatives of 5-(dipentylamino)-5-oxo-pentanoic acid have also been found to have a very good CCK-antagonistic effect. Furthermore, various synthetic peptide derivatives of CCK and its fragments e.g. t-butyloxycarbonyl-Tyr(SO₃)-Met-Gly-D-Trp-Nle-Asp-2-phenylethyl ester, CCK 27-33 and even peptide derivatives of substance P developed for blocking substance P receptors, e.g. (D-Pro₄,D-Trp_{7,9,10})-substance P4-11, have been found to have a CCK-blocking effect. It is moreover known from the literature that esters of beta-carbolines such as methyl or ethyl beta-carboline-3-carboxylate may exert an inhibitory effect on CCK. A fact that is very interesting is that there are various benzodiazepine derivatives such as e.g. 3-substituted 1,4-benzodiazepine-2-amines and 4-substituted 4H-(1,2,4)-triazolo(4,3-a)(1-4)-benzodiazepines which have an inhibitory effect on CCK. Benzodiazepines such as chlordiazepoxide, diazepam and medazepam may also be active. It has also been reported recently that substances deriving from Aspergillus alliaceus, which are called "Asperlicins", are CCK receptor inhibitors. Starting from this group of substances additional potent derivatives have been prepared which exhibit a strong CCK receptor antagonistic effect. One of these substances is 3S(-)-N-(2,3-dihydro-1-methyl-2-oxo-5-phenyl-1H-1,4-benzodiazepine-3-yl)-1H-indole-2-carboxamide, also called L-364,718. This substance is one of the most efficient CCK inhibitors hitherto known. Other inhibitors of CCK also mentioned in the literature are benzotript and CR-1392, and A-64718.

Among the aforesaid substances may be mentioned expecially the following antagonists: Lorglumide (CR 1409) (Makovec et al., 1987a; 1987b), L-364,718 (Lotti et al., 1987), Proglumide Loxiglumide (CR-1505) and N(4-chlorobenzoyl)-L-tryptophan.

Low toxicity and good bioavailability have been reported as properties of several cholecystokinin antagonists. More such substances may be expected to come forth, with additional useful properties.

Substances to be used according to the present invention are previously known to be pharmaceutically active. So has for instance the octapeptide of CCK been utilized for emptying the gallbladder.

There are several situations where a miosis is desirable - for example after a lens implantation has been made if the pupil width is then too great, or after examination of the fundus of eye under cholinergic blockade. A possibility to then bring about miosis with the aid of CCK or fragments thereof appears to be a very attractive concept. Local administration directly into the aqueous humor when surgery is being performed, topically on the cornea, or subconjunctivally, may be expected to produce the effect desired.

The present invention thus relates to cholecystokinin, and derivatives and analogues of this peptide, especially its C-terminal portion, for inducing miosis in the eye after certain types of examinations and intraocular operations. Only pharmaceutically active and physiologically acceptable CCK derivatives and analogues are of course intended to be used according to this invention.

Furthermore, the invention comprises compositions containing an effective amount of cholecystokinin or derivatives or analogues of this peptide in an ophthalmologically compatible vehicle for inducing miosis after certain types of examinations and intraocular surgery. The term "effective amount" here means that the composition contains from 10 pg to 100 µg thereof, depending on whether it is introduced directly into the anterior chamber in connection with the operational procedure, or whether it is applied topically or applied subconjunctivally.

The ophthalmologically compatible vehicle that may be employed for preparing compositions according to this invention consists of aqueous solutions such as for example physiological salines for compositions to be inserted into the eye, e.g. into the anterior chamber or subconjunctivally.

The ophthalmologically compatible vehicle that may be employed for preparing compositions for topical use consists of aqueous solutions such as for instance physiological salines, oil solutions or ointments. Furthermore the vehicle may contain - especially in cases where the composition is intended for topical use - ophthalmologically compatible preservatives such as e.g. benzalkonium chloride, surfactants, liposomes or polymers, e.g. methyl cellulose, polyvinyl alcohol, polyvinyl pyrrolidone, hyaluronic acid, which may be employed for the purpose of increasing viscosity. Also soluble and insoluble drug inserts may be included in the vehicle when the cholecystokinin, its derivatives or analogues, or antagonists, are to be administered topically.

The invention moreover also relates to a method of inducing miosis after certain types of examinations and intraocular surgery. The method consists in administration of a therapeutically active amount of a composition containing at least one of the substances defined above. In a preferred embodiment a composition as described above is contacted with the eye so as to either induce or prevent miosis. The composition contains cholecystokinin or derivatives or analogues of this substance for inducing miosis.

The invention is illustrated by a series of non-limitating examples; results are set forth in Figures 1-7 as follows:
- Figure 1:: The effect of injection of CCK-8 on pupil size in three monkey eyes.
- Figure 2:: The effect on pupil size when CCK-8 is applied on the cornea.
- Figure 3A:: Cumulated dose-response curve of cholecystokinin on monkey iris in vitro.
- Figure 3B:: Cumulated dose-response curve of the C-terminal octapeptide (26-33) of cholecystokinin on monkey iris in vitro.
- Figure 4A:: Cumulated dose-response curve of cholecystokinin on monkey iris in vitro in the presence and absence of Proglumide, a CCK receptor antagonist.
- Figure 4B:: Cumulated dose-response curve of CCK on monkey iris in vitro in the presence and absence of N(4-chlorobenzoyl)-L-tryptophan, a CCK receptor antagonist.
- Figure 4C:: Cumulated dose-response curve of cholecystokinin on monkey iris in vitro in the presence and absence of Lorglumide, a CCK receptor antagonist.
- Figure 4D:: Cumulated dose-response curve of the C-terminal octapeptide (26-33) of cholecystokinin on monkey iris in vitro in the presence and absence of Proglumide.
- Figure 4E:: Cumulated dose-response curve of the C-terminal octapeptide (26-33) of cholecystokinin on monkey iris in vitro in the presence and absence of N(4-cholobenzoyl)-L-tryptophan.
- Figure 4F:: Cumulated dose-response curve of the C-terminal octapeptide (26-33) of cholecystokinin on monkey iris in vitro in the presence and absence of Lorglumide.
- Figure 5:: Dose-response curve of CCK-8 with and without pretreatment with the antagonist Lorglumide in the monkey eye in vivo.
- Figure 6:: Cumulated dose-response curve of the C-terminal octapeptide of CCK on the sphincter muscle of human iris in vitro.
- Figure 7:: The effect of Capsaicin on pupil size with and without pretreatment with 7.5 µg Lorglumide.

### Experiments

Example 1: Monkeys employed in these experiments (Macaca fascicularis) were anesthesized with pentobarbital. The anterior chambers of the eyes were cannulated with 2 special needles each. Substance could be injected through one of the needles which was connected via a polyethylene tube to a syringe for small volumes (100µl). A corresponding volume of aqueous humor could be drawn off via the other needle to thus avoid eye pressure alterations due to the intracameral injection of the test substances. The test substance, CCK or CCK-8, was dissolved in isotonic saline.

The animals were first treated with atropine to induce maximal dilatation of the pupil. The dose-response relationship was then determined for one eye. Pupil size was recorded as obtained by measuring the horizontal diameter of the pupil by means of a graduated ruler, these measurements being made at regular intervals and under standard light conditions. Typical examples of these experiments are shown in Figure 1.

Example 2: Atropine treated monkeys which had been anesthesized with pentobarbital were given every ten minutes a drop of 6-12 µl of a solution containing 250 ng/µl CCK-8, on the cornea of the left eye. This was carried on during a time span until 100 minutes from start; at that stage 100 µl were administered, followed by 10 µl every ten minutes. 60 minutes after start, pupil size decrease was observed in the treated eye. The pupil then went on decreasing during the course of the experiment (Figure 2). No effect was seen in the untreated control eye.

Example 3: Iris tissues from monkeys (Macaca fascicularis) were obtained immediately after the animals had been sacrificed. The tissues were transported in saline on ice and were mounted in a conventional muscle bath system. Increase of tension in the tissue, which was held in a fixed position at each end of the cut muscle, was measured isometrically by means of Grass Transducers coupled to a Grass 7D polygraph and was expressed as mm recordings on the polygraph. The pieces of tissue were lying immersed in a bath consisting of a standard Ringer solution for muscle baths, with continuous oxygen supply and controlled temperature (35°). Indomethacin, propranolol and atropine were added to the solution in order to eliminate the effects of prostaglandin, beta-adrenergic receptors and muscarinic receptors. Varying amounts of CCK, CCK-8 dissolved in isotonic saline were added to the bath, and the cumulated dose-response curves obtained with CCK and CCK-8 were drawn up. These curves are illustrated in Figure 3. With the tetrapeptide of the C-terminal portion of CCK, 30-33, no muscle contraction was obtained - not even with a total dose of 86 µg dispersed in the 10 ml tissue bath.

Example 4: When the cumulated dose-response curves had been obtained the preparations were carefully rinsed and one of three antagonists, viz., Lorglumide or Proglumide or N(4-chlorobenzyl)-1-tryptophan, was added to the baths. About 10 to 20 minutes later a corresponding cumulated dose-response curve was drawn up for CCK and CCK-8 in the presence of one of the antagonists in the bath. After completion of this dose-response curve in the presence of antagonist the baths again were carefully rinsed; then again a specified amount of CCK or CCK-8 was tested without the presence of an antagonist in order to thus investigate reversibility properties. Figure 4 illustrates the results obtained in experiments with the aforesaid three antagonists to CCK and CCK-8 on in vitro monkey iris. The volume of the tissue baths was 10 ml.

Example 5: Dose-response determination of CCK-8 was carried out on one eye of atropine treated, pentobarbital anesthesized monkeys in accordance with the description in Example 1. Thereafter 0.75µg of Lorglumide in 15µl of isotonic saline was injected into the anterior chamber of the other eye. The CCK-8 dose-response relationship was then determined in the same way as in the case of the first eye. The CCK antagonist produced a shift of the dose-response curve by more than one order of magnitude (Figure 5).

Example 6: Iris tissue from an enucleated human eye was obtained immediately after operation. The sphincter muscle was isolated and mounted in a tissue bath as has been described in Example 3. A cumulated dose-response curve for CCK-8 was drawn up in a manner analogous to that described in Example 3. The result is illustrated in Figure 6.

Example 7: Monkeys (Macaca fascicularis) were anesthesized, pretreated with atropine and cannulated as described in Example 1. Then 10 µl of a 1% solution of Capsaicin was injected into the anterior chamber; the diameter of the pupil was measured 15 minutes later. The decrease in pupil size obtained with this dose varied from 0 to 1.5 mm. Animals that had been found to be sensitive to Capsaicin in one eye were later injected with the same dose of Capsaicin in the other eye after pretreatment of that other eye with Lorglumide, 7.5 µg in 15 µl. The CCK antagonist decreased the response to Capsaicin (Figure 7).

Our experiments thus show that cholecystokinin or its octapeptide, and probably some closely related derivatives as well, induce miosis in primates (including humans) by stimulating cholecystokinin receptors in the iris. Selective blockade of said receptors, using any of a variety of antagonists, will counteract this miosis. (It also appears that the blockage of CCK receptors prior to irritative stimuli will diminish irritation miosis in the monkey.) Consequently it seems probable that the mechanism responsible for irritation miosis in primates resides in a release of CCK or a closely related substance which produces miosis by way of stimulating CCK receptors on smooth muscle cells in the iris sphincter muscle. This miosis is independent of acetylcholine.

The above examples show that CCK or closely related substances acting upon CCK receptors can be utilized for inducing miosis for therapeutical purposes, e.g. after cataract surgery with implantation of an artificial lens, and inversely, antagonists to CCK or closely related substances acting through the same receptors can be used for preventing miosis during the operation itself, or they may be utilized for inhibiting such miosis as will occur in association with iritis, uveitis and trauma.

### REFERENCES

Bill, A. Stjernschantz, J., Mandahl, A., Brodin, E. & Nilsson, G. 1979. Substance P: Release on trigeminal nerve stimulation, effects in the eye. Acta Physiol Scand 106:371-373.

Kuwayama, Y., Terenghi, G., Polak, J.M., Trojanowski, J.Q. & Stone, R.A. 1987. A quantitative correlation of substance P-, calcitonin gene-related peptide- and cholecystokininlike immunoreactivity with retrogradely labelled trigeminal ganglion cells innervating the eye. Brain Res. 405:220-226.

Lotti, V.J., Pendleton, R.G., Gould, R.J., Hanson, H.M., Chang, R.S.L. & Clineshmidt, B.V. 1987. In vivo pharmacology of L-364, 718, a new potent non-peptide peripherial cholecystokinin antagonist. J Pharmacol and Exp Therap. 241:103-109.

Makovec, F., Bani, M., Cereda, R., Chiste', R., Pacini, M.A., Revel, L., Rovati, L.C. & Setnikar, I. 1987a. Pharmacological properties of lorglumide as member of a new class of cholecystokinin antagonists. Arzneim.forsch./Drug Res. 37(11), No. 11, pp. 1265-1268.

Makovec, F., Bani, M., Cereda, R., Chiste', R., Pacini, .A., Revel, L. & Rovati, L.C. 1987b. Antispasmodic activity on the gallbladder of the mouse of CR 1409 (lorglumide) a potent antagonist of peripheral CCK. Pharmacol Res Comm. 19:41-51.

Mandahl, A., Brodin, E., Nilsson, G. & Bill, A. 1980. Substance P, release and effects in the eye. Acta Physiol Scand. 108:18A.

## Claims

1. Use of cholecystokinin, its C-terminal octapeptide, other pharmaceutically active and physiologically acceptable derivatives and analogues for the manufacturing of a composition for inducing pupil constriction in the eye.

2. Use of the C-terminal octapeptide according to claim 1.

3. Use according to anyone of claims 1-2 wherein the composition contains 10 pg to 100 µg of cholecystokinin, its C-terminal octapeptide or a pharmaceutically active and physiologically acceptable derivative or an analogue.

4. Ophthalmological composition for the control of pupil constriction, characterized in that it is adapted for ophthalmological administration and comprises a therapeutically active amount of cholecystokinin, its C-terminal octapeptide or a pharmaceutically active and physiologically acceptable derivative or an analogue, in an ophthalmologically compatible vehicle.

5. Ophthalmological composition according to claim 4, characterized in that the ophthalmologically compatible vehicle is a physiological salt solution, an oil solution or ointment, and optionally contains also ophthalmologically compatible preservatives, surfactants, liposomes or polymers.
